# EUROPEAN PATENT APPLICATION

(11) **EP 4 292 426 A1**
(43) Date of publication of application: **20.12.2023**
(21) Application number: 23179142.7
(22) Date of filing: 14.06.2023
(51) Int. Cl.: A01H 6/46, A01H 5/06, A01H 5/04, A01H 4/00

(54) **JUVENILE BAMBOO PLANT OF THE GENUS FARGESIA**

(30) Priority: 15.06.2022 BE 202205473
(71) Applicant: Plant Select NV, 2310 Rijkevorsel (BE)
(72) Inventor: PEETERS, Hilde, 2310 Rijkevorsel (BE)
(74) Representative: Brantsandpatents bv

(57) **Abstract**

The present invention relates to a method for producing a juvenile bamboo plant of the genus Fargesia, wherein the plant has a pachymorph root system, and wherein the plant, at an age of between 4 and 6 months, has between 20 and 70 stems, which are substantially between 2 and 7 cm long, wherein the juvenile bamboo plant has been obtained by means of an in vitro micropropagation method.

## Description

### TECHNICAL FIELD

The invention relates to a new juvenile bamboo plant of the genus Fargesia, and a method for its production.

### PRIOR ART

The subfamily Bambusoideae (Poaceae) includes both woody and herbaceous bamboos, but woody bamboos especially have economic potential. Bamboos are versatile plants with many different uses. The demand for bamboo is increasing, primarily due to the serious depletion of the forests and the possibilities of using bamboo as a substitute for tropical wood.

Fargesia is a genus of medium-sized bamboo. Fargesia species are bamboos that grow in clumps and do not form rhizomes (runners) like most bamboos do. The species are well usable in small gardens without nuisance from runners that lift tiles or plants growing in unwanted places. Due to the compactness of the plant and the clump-shaped growth, Fargesia is increasingly used as a partition or hedge. Due to the variety of species, Fargesia can be obtained in different growth forms from 1.25 high to 4 meters high.

However, growing and propagating bamboo species with a lot of biomass is made more difficult by contamination with microorganisms. Many, if not all, naturally grown and propagated plants are therefore endogenously contaminated with microorganisms of various kinds, which leads to a loss of biomass. In addition, bamboo producers lose a second time by preventing and treating these infections, which is very time and resource intensive.

Propagating bamboo plants today is mainly done by tearing existing bamboo plants. The plants are literally torn apart at the level of the roots to obtain two plants. The disadvantage here is that the plants are or become mature after a certain time. This is not favorable if a lot of biomass is desired. In contrast, juvenile - non-mature - plants have the advantage of young tissue that grows easily.

In addition, bamboo is also propagated via in vitro micropropagation techniques, as discussed in Gielis Johan, et al., "Micropropagation of temperate and tropical wooding bamboos - From biotechnological dream to commercial reality", 26 July 2002; R. L. Baniak, et al., "Propagation and management", 22 June 1995; Konzen, et al., "Management of bamboo genetic resources and clonal production systems", 30 August 2021; and D4: Jimemez Victor M., et al., "Standard protocols for in vitro propagation of bamboo with emphasis on axillary shoot proliferation", 30 August 2021.

Although these techniques proved to be efficient for the propagation of bamboo, this bamboo also remains susceptible to contamination with microorganisms.

Thus, there is a need for an improved and compact Fargesia plant with a lot of biomass, which remains juvenile longer and is relatively free of pathogens, as well as methods for its production.

The invention therefore aims to provide a solution to at least some of the above problems.

### SUMMARY OF THE INVENTION

The invention relates to a method for producing a juvenile bamboo plant of the genus Fargesia according to claim 1. The plant has a pachymorph root system, and at an age of between 4 and 6 months, the plant has between 20 and 70 stems, which are substantially between 2 and 7 cm long, whereby the juvenile bamboo plant has been obtained by means of an in vitro micropropagation method as described in claim 1.

Such bamboo plant of the present invention is a very strong plant and grower which is relatively germ free. In addition, such a bamboo plant remains much longer in the juvenile stage, so that more young tissue and more stems are present. The bamboo plant is also more compact than other bamboo plants of the Fargesia species.

Preferred embodiments of the method are described in claims 2-11.

### DESCRIPTION OF THE FIGURES

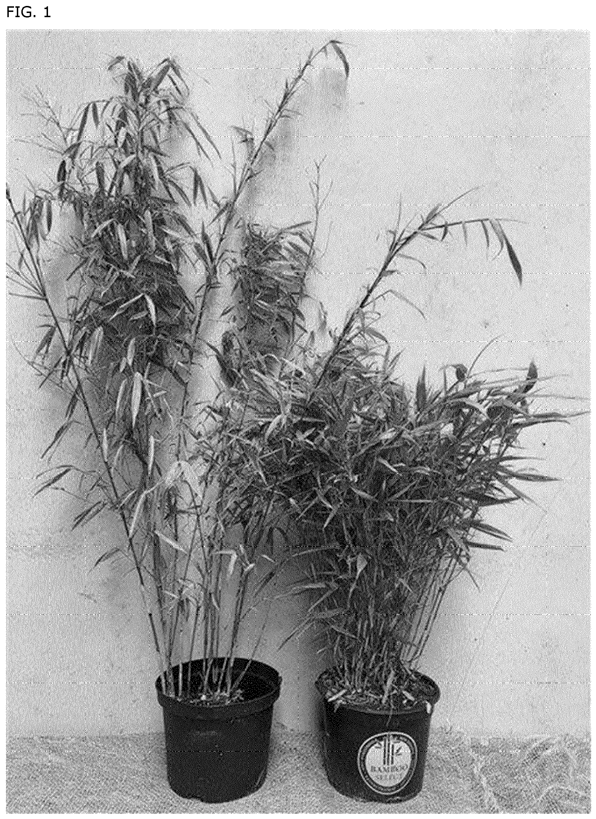

**Figure 1** shows a comparison between a *Fargesia rufa* plant obtained by the traditionally used technique of "tearing" (left) and a *Fargesia rufa* plant according to an embodiment of the present invention obtained by an in vitro propagation method (right).

### DETAILED DESCRIPTION

The invention relates to a juvenile bamboo plant of the genus Fargesia, the plant having, at an age of between 4 and 6 months, between 20 and 70 stems, which are substantially between 2 and 7 cm long, the juvenile bamboo plant being obtained by means of an in vitro micropropagation method.

Unless otherwise defined, all terms used in the description of the invention, including technical and scientific terms, have the meanings commonly understood by those skilled in the art of the invention. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise," "comprising," "consist of," "consisting of," "provided with," "have," "having," "include," "including," "contain," "containing" are synonyms and are inclusive or open terms that indicate the presence of what follows, and which do not exclude or prevent the presence of other components, characteristics, elements, members, steps, as known from or disclosed in the prior art.

Quoting numeric intervals by the endpoints includes all integers, fractions, and/or real numbers between the endpoints, including those endpoints.

In a first aspect, the invention relates to a juvenile bamboo plant of the genus Fargesia, wherein the plant has a pachymorph root system, and wherein the plant, at an age of between 4 and 6 months, has between 20 and 70 stems, which are substantially between 2 and 7 cm long, wherein the juvenile bamboo plant has been obtained by means of an in vitro micropropagation method. In an embodiment, at an age of between 4 and 6 months, this plant has between 15 and 70 stems, or between 20 and 70 stems, or between 30 and 70 stems, which are substantially between 2 and 7 cm long.

"Juvenile" plants are plants that are in an early phase of plant growth, where the plant grows larger but is not yet able to reproduce. In other words, a juvenile plant has not yet bloomed. The term "mature," "fully grown" or "adult" is opposed to "juvenile." The biomass of a juvenile plant increases more than compared to a mature plant with otherwise very similar characteristics.

One of the advantages of the juvenile bamboo plant of the present invention obtained by an in vitro micropropagation method is that it is a very strong plant and grower with a lot of biomass, and relatively free from pathogens. In addition, such a bamboo plant remains much longer in the juvenile stage, so that more young tissue and more stems are present. Many new juvenile bamboo plants can be obtained in a short time by means of the in vitro propagation method. On the other hand, there are bamboo plants which have been obtained by tearing existing bamboo plants. These were not propagated via such an in vitro method, do not remain juveniles for as long and are also less pathogen-free. In addition, plants obtained by means of the in vitro propagation method are also more uniform among themselves, which is an added value in further propagation since the plants need an equal amount of water and nutrients.

In an embodiment, the bamboo plant of the present invention is a Fargesia species. This can be any Fargesia species, for example, but not limited to Fargesia acuticontracta, Fargesia adpressa, Fargesia alatovaginata, Fargesia albocerea, Fargesia altior, Fargesia angustissima, Fargesia apicirubens, Fargesia brevipes, Fargesia brevissima, Fargesia caduca, Fargesia canaliculate, Fargesia circinate, Fargesia communis, Fargesia concinna, Fargesia conferta, Fargesia contracta, Fargesia cuspidate, Fargesia daminiu, Fargesia declivis, Fargesia decurvata, Fargesia denudate, Fargesia dracocephala, Fargesia dulcicula, Fargesia dura, Fargesia edulis, Fargesia elegans, Fargesia exposita, Fargesia extensa, Fargesia fansipanensis, Fargesia farcta, Fargesia ferax, Fargesia frigidis, Fargesia fungosa, Fargesia funiushanensis, Fargesia glabrifolia, Fargesia gongshanensis, Fargesia grossa, Fargesia hackelii, Fargesia hainanensis, Fargesia hsuehiana, Fargesia hygrophila, Fargesia jiulongensis, Fargesia lincangensis, Fargesia longiuscula, Fargesia lushuiensis, Fargesia macclureana, Fargesia mairei, Fargesia mali, Fargesia melanostachys, Fargesia microauriculata, Fargesia murielae, Fargesia nitida, Fargesia nivalis, Fargesia nujiangensis, Fargesia obliqua, Fargesia orbiculate, Fargesia papyrifera, Fargesia pauciflora, Fargesia perlonga, Fargesia pleniculmis, Fargesia plurisetosa, Fargesia porphyria, Fargesia praecipua, Fargesia qinlingensis, Fargesia robusta, Fargesia rufa, Fargesia sagittatinea, Fargesia scabrida, Fargesia semicoriacea, Fargesia similaris, Fargesia solida, Fargesia spathacea, Fargesia stenoclada, Fargesia strigose, Fargesia subflexuosa, Fargesia sylvestris, Fargesia tengchongensis, Fargesia tenuilignea, Fargesia ungulate, Fargesia utilis, Fargesia vicina, Fargesia weiningensis, Fargesia wuliangshanensis, Fargesia yajiangensis, Fargesia yuanjiangensis, Fargesia yulongshanensis, Fargesia yunnanensis, and Fargesia zayuensis. The bamboo plant is preferably selected from the Fargesia species Fargesia adpressa, Fargesia angustissima, Fargesia dracocephala, Fargesia murielae, Fargesia nitida, Fargesia scabrida and Fargesia yunnanensis.

In another or further embodiment, the bamboo plant of the invention is a Fargesia species selected from the group of Fargesia dracocephala "Red Dragon," Fargesia papyrifera "Blue Dragon," Fargesia demissa "Black Dragon," Fargesia scabrida "Asian Wonder," Fargesia rufa "Rufa," Fargesia robusta "Pingwu," Fargesia robusta "Formidable," Fargesia nitida "Gansu" and Fargesia angustissima "Angustissima."

According to an embodiment, the juvenile bamboo plant of the invention is provided in a substrate, preferably a glue plug-based substrate. Such glue plug-based substrate has a high volume of air. This is important to enable good root formation in the substrate. Fargesia species have a harder time than some other species to form roots in a dense substrate or in dense nutrient media. By using this glue plug-based substrate, firm roots are formed more quickly, which also has a clear effect above ground. The number of stems of a juvenile bamboo plant provided in a glue plug-based substrate is higher, as well as the total volume of shoot material. Ideally, a glue plug retains an air content of at least 20%, preferably at least 25%, more preferably at least 30% air content within the volume of the plug, even if the plug is heavily misted.

According to an embodiment, the glue plug-based substrate comprises coconut peat and a binder, and optionally perlite and/or peat. The glue plug may further comprise fertilizers such as NPK fertilizers, N fertilizers and/or iron.

According to an embodiment, the juvenile bamboo plant of the invention is provided in the glue plug-based substrate watered with redox water. In a further embodiment, the plant is watered with redox water at least twice a month, preferably at least 3 times a month, preferably at least 4 times a month. In another or further embodiment, the plant is watered every week with redox water, sometimes up to 2 times a week. Preferably, the plants are watered in between with rainwater or tap water, possibly supplemented with nutrients.

"Redox water" is water with a high redox potential and kills bacteria, fungi and even viruses. By using redox water, the number of pathogens is further kept low. This is important as bamboo is very susceptible to various plant diseases. Among other things, this prevents infection with Botrytis, one of the largest pathogens in horticulture. Preferably, this redox water comprises hypochlorous acid (HClO) and hypochlorite ion (OCl), and this redox water has a redox potential of between 700 and 900 mV, depending on the water quality.

More specifically, watering with redox water also has the specific advantage that endogenous contamination is tackled. Bamboos are among the most "dirty" plants of all and are almost always endogenously contaminated with microorganisms of various kinds. In the past, fungicides or antibiotics were used in tissue culture plants to tackle diseases, and/or it was opted to sterilize the surface of the (ex)plants or seeds using, for example, acetone followed by a rinsing phase in sterile water. These methods were not sufficiently efficient, or only led to a postponement of the problems to a later stage in the growth and development of the plant. Contamination of bamboo plants usually also occurred at a later stage of development, for example via the air. The plants and cultures therefore had to be constantly tested and visually inspected to detect and act on the first signs of contamination. The extra cost of this monitoring, which could amount to 20% of the operator's time, could easily be recouped by preventing contamination in the laboratory followed by a method to prevent later contamination. To prevent (air) contamination, it was previously already decided to only water the plants in the pot in which they grow, and not to spray the plants themselves. However, the current method in which plants, preferably in the pot, are regularly watered with redox water, proved to be even more efficient in preventing contamination and infection.

The juvenile bamboo plant of the present invention is more compact than plants obtained by the traditional "tearing" of bamboo plants. For example, the stems are shorter and these shorter stems carry more leaf material. In addition, this compactness is also reflected in the number of leaves on the stems low against the substrate, which is much higher than with such traditional bamboo plants.

According to an embodiment of the current invention, the juvenile bamboo plant at an age of 18 months has at least 2, preferably at least 3, preferably at least 4, preferably at least 5, preferably at least 6, preferably at least 7, preferably at least 8, preferably at least 9, preferably at least 10, preferably at least 11, preferably at least 12 leaves on the stems over a length of 10 cm measured from the substrate along the stems.

According to an embodiment of the present invention, at 18 months of age the juvenile bamboo plant has at least 27, preferably at least 28, preferably at least 29, preferably at least 30, preferably at least 31, preferably at least 32, preferably at least 33, preferably at least 34, preferably at least 35, preferably at least 36, preferably at least 37, preferably at least 38, preferably at least 39, preferably at least 40, preferably at least 41, preferably at least 42, preferably at least 43, preferably at least 44, preferably at least 45 leaves on the stems over a length of 20 cm measured from the substrate along the stems.

According to an embodiment of the present invention, the juvenile bamboo plant is obtained by an in vitro micropropagation method from bamboo stem explants via direct shoot propagation, more specifically via axillary branching.

"Direct micropropagation" is the rapid multiplication of plant material to produce a large number of plant progeny using modern plant tissue culture methods. If starting from shoot material, this is called "direct shoot propagation." "Axillary branching" is a direct micropropagation method starting from shoot material, specifically where axillary buds formed on that shoot are stimulated to rapidly grow into further shoots. This process can be repeated several times and the initial shoot can be transformed into a mass of branches.

Preferably, the in vitro micropropagation method, by which the juvenile bamboo plant of the invention is obtained, comprises the following steps:
a. disinfecting the bamboo stem explants using redox water,
b. propagating the stem explants on propagation medium, and
c. inducing root formation on a rooting medium to a rooted explant,
after which the rooted explant is transplanted into a glue plug-based substrate, the substrate substantially consisting of peat and a binding agent, and after which the explant is grown in substrate in a greenhouse or grow room into a juvenile bamboo plant.

As already described above, the redox water kills bacteria, fungi and even viruses. By making the stem explants aseptic with the redox water, the number of pathogens is kept low from the start of the bamboo plant propagation. Preferably, this redox water comprises hypochlorous acid (HClO) and hypochlorite ion (OCl), and this redox water has a redox potential of between 700 and 900 mV, depending on the water quality. Internal comparative tests have unexpectedly shown that this disinfection step yields better results and disinfection with other known disinfectants suitable for plants or, for example, disinfection using ethanol or acetone.

The "propagation medium" in the present context is a medium in which shoots are propagated, more specifically a medium suitable for inducing axillary branching.

In an embodiment, the propagation medium is an MS medium supplemented with one or more auxins and one or more cytokinins. These auxins and cytokinins can be any kind of natural or synthetic auxins or cytokinins known to one of ordinary skill in the art. Preferably, these cytokinins are selected from the group consisting of N6(Δ2-isopentenyl)adenine, trans-zeatin, dihydrozeatin, N6(benzyl)adenine, N6(benzyl)adenosine, 6-furfurylaminopurine, and thidiazuron.

In a further embodiment, the cytokinins other than thidiazuron are present in the propagation medium at a concentration of 0.5-10 mg/L, and/or thidiazuron is present in the propagation medium at a concentration of 0.01-0.5 mg/L.

In an embodiment, the explant remains on propagation medium for about 5 weeks.

The "rooting medium" in the present context is a medium in or through which the induction of the formation of roots on the explants is stimulated.

In an embodiment, the rooting medium is an MS medium comprising auxins. These auxins can be selected from all types of natural or synthetic auxins known to a person skilled in the art. Preferably, these auxins are present in a concentration of 0.1-5 mg/L. Preferably, the auxins are selected from naphthalene acetic acid (NAA) or indole butyric acid (IBA).

In an embodiment, the rooting medium further comprises KH₂PO₄ as a phosphorus and potassium source, inositol and a sugar.

Phosphorus (P) is a macronutrient and is essential for plant growth. Phosphorus plays an important role in growth and development, such as in cell division, albumin formation, and callus development. In addition to phosphorus, potassium (K) also plays an important role in plant growth and development. Elemental Potassium (K) has functions such as activating enzymes, opening stomata and embryonic development.

Myo-inositol (inositol) is a sugary carbohydrate produced by most plants. Myo-inositol (inositol) is important for normal plant growth and development, and is required for phosphate storage, cell wall biosynthesis, production of stress-related molecules, cell-to-cell communication, and for storage and transport of plant hormones.

A sugar, such as glucose, affects the growth of plants and causes delays in development from the juvenile to the vegetative phase. Glucose induces the synthesis of chlorophyll, rubisco and various photoprotective pigments. Glucose reduces the harmful effects of abiotic stress by increasing antioxidant and sugar levels. For example, the explant grown on rooting medium can be grown in a closed jar. Such plants in a closed jar cannot absorb CO₂ from the air outside the jar. In that case, the sugar in the medium is also very important as a carbon source for the explant.

In an embodiment, the propagation medium and/or rooting medium is hardened with a gelling agent, preferably based on gellan gum. Preferably gellan gum gelling agent is present at a concentration of 1.6-2 g/L in the medium.

In an embodiment, the propagation medium and/or rooting medium further comprises adenine hemisulfate, biotin and folic acid.

Adenine hemisulfate as a component in a plant growing medium improves the frequency of multiple shoot production.

Biotin is an essential vitamin important for amino acid and energy metabolism, as well as for the synthesis of fatty acids. Namely, it acts as a cofactor for a series of enzymes that catalyze carboxylation, decarboxylation and transcarboxylation reactions in a number of crucial metabolic processes.

Folic acid and other folates are involved in the synthesis of amino acids and nucleic acids. Furthermore, folic acid enables plants to metabolize carbohydrates, proteins and lipids. Although folic acid occurs naturally in many plants, it is also quickly broken down when exposed to strong light. This makes it advantageous to add an additional amount of folic acid to the plant medium.

In an embodiment, the explant remains on rooting medium for about 5 weeks.

After inducing root formation, the rooted explant is transplanted into a glue plug-based substrate. Said substrate is preferably a substrate as already described above and consists substantially of peat and a binder. The rooted explant is transplanted into the glue plug-based substrate when the plant is approximately 10 weeks old (after approximately 5 weeks on propagation medium and approximately 5 weeks on rooting medium).

After transplanting into such substrate, the explant is grown in substrate into a juvenile bamboo plant in a greenhouse or grow room.

Preferably, the plant is grown in the greenhouse or grow room under relatively moist conditions, preferably under plastic or mist, to guarantee high humidity.

Preferably, the rooted explant in the substrate and/or the juvenile bamboo plant in the greenhouse or grow room is watered with redox water instead of plain water, as already indicated above.

In what follows, the invention is described by way of non-limiting examples illustrating the invention, and which are not intended to and should not be interpreted as limiting the scope of the invention.

### EXAMPLES

### Example 1: production of the juvenile Fargesia bamboo plant according to an embodiment of the invention

Stem explants are propagated by axillary branching. For this purpose, the stem explants are first disinfected using redox water containing hypochlorous acid (HClO) and hypochlorite ion (OCl), and with a redox potential of between 700 and 900 mV.

The stem explants are then placed on MS-based propagation medium supplemented with auxins and cytokinins, where the cytokinins are selected from the group consisting of N6(Δ2-isopentenyl)adenine, trans-zeatin, dihydrozeatin, N6(benzyl)adenine, N6(benzyl)adenosine, 6-furfurylaminopurine, and thidiazuron. Tiadiazuron is present in the medium in a concentration of 0.01-0.5 mg/L, and the other cytokinins in a concentration of 0.5-10 mg/L. The propagation medium further comprises gellan gum at a concentration of 1.6-2 g/L, as well as adenine hemisulfate, biotin and folic acid. The stem explants remain on the propagation medium for approximately 5 weeks.

As a result, the stem explants develop shoots very efficiently.

Subsequently, the stem explants with shoots are moved to a rooting medium which stimulates the induction of root formation on the explants. This medium is an MS medium to which auxins are added, more specifically to which naphthalene acetic acid (NAA) or indole butyric acid (IBA) is added in a concentration of 0.1-5 mg/L. The rooting medium further comprises KH₂PO₄, inositol and a sugar; as well as gellan gum at a concentration of 1.6-2 g/L, adenine hemisulfate, biotin and folic acid. The stem explants remain on the rooting medium for about 5 weeks.

After inducing root formation, the rooted explant is transplanted into a glue plug-based substrate, for example based on coconut peat, perlite and peat, in which a high volume of air is present, allowing strong roots to be formed. At transplanting, the explant is approximately 10 weeks old (5 weeks on propagation medium and 5 weeks on rooting medium). After transplanting into such a substrate, the explant is grown in the substrate into a juvenile bamboo plant in a greenhouse or grow room under relatively humid conditions, such as grown under plastic, to ensure high humidity.

The rooted explant in substrate and later the juvenile bamboo plant in the greenhouse or grow room is watered once a week with redox water comprising hypochlorous acid (HClO) and hypochlorite ion (OCl), and with a redox potential of between 700 and 900 mV, to prevent contamination. In between, the plant is watered with rainwater.

At an age of between 4 and 6 months, the obtained juvenile bamboo plants have between 20 and 70 stems, which are substantially between 2 and 7 cm long. These plants are relatively free of pathogens.

For example, three juvenile bamboo plants of the present invention were grown for 10 weeks on propagation and rooting medium, after which they were grown for 3 months in substrate. Plant 1 had 61 stems between 2.5 and 6.5 cm long, Plant 2 had 32 stems between 2 and 6.5 cm long, and Plant 3 had 63 stems between 3 and 6 cm long. All three plants are compact, extremely free from endogenous contamination, strong growers and remain juveniles for a long time.

### Example 2: comparison between a Fargesia rufa plant obtained in a traditional way and a Fargesia rufa plant according to an embodiment of the present invention

**Figure 1** shows a comparison between a *Fargesia rufa* plant obtained by the traditionally used technique of "tearing" (left) or a *Fargesia rufa* plant according to an embodiment of the present invention obtained by an in vitro propagation method (right). Both plants are about 18 months old.

| **Feature** | **Plant traditionally torn apart** | **Juvenile plant according to an embodiment of the present invention** |
|---|---|---|
| Mature/juvenile | Mature | Juvenile |
| Number of stems/culms with branching | 16 | 63 |
| Length stems (cm) | 93 | 55 |
| Number of leaves on the stems over a length of 10 cm measured from the substrate along the stems | 0 | 14 |
| Number of leaves on the stems over a length of 20 cm measured from the substrate along the stems | 35 | 48 |

This clearly shows that the bamboo plant of the present invention remains juvenile longer, is more compact, forms more stems and shows more leaf formation already from the bottom of the stems and the plant as a whole.

In plants less than 18 months old, this compactness is even more pronounced compared to traditionally torn apart plants. For example, the length of the stems of a traditionally torn apart plant at 5 months of age may be about 12 cm, while the length of plants according to the present invention is about 6 cm at that age. Another example is a traditionally torn apart plant at 10 months of age which has stems of about 60 cm, while a plant according to the present invention has stems of about 30 cm at that age.

The present invention should not be construed as being limited to the embodiments described above and certain modifications or changes may be added to the examples described without having to re-evaluate the appended claims.

## Claims

1. A method of producing a juvenile bamboo plant of the genus Fargesia provided in a glue plug-based substrate, the plant having a pachymorph root system, the plant having between 20 and 70 stems at an age of between 4 and 6 months, which stems are substantially between 2 and 7 cm in length, where the juvenile bamboo plant has been obtained by an in vitro micropropagation method from bamboo stem explants via direct shoot propagation, in particular via axillary branching, where the in vitro method comprises the following steps:
a. disinfecting the stem explants using redox water,
b. propagating the stem explants on propagation medium, and
c. inducing root formation on a rooting medium to a rooted explant,
d. transplanting the rooted explant into a glue plug-based substrate,
e. growing the explant in substrate in a greenhouse or grow room into a juvenile bamboo plant,
wherein the juvenile bamboo plant in the substrate is watered with redox water at least twice a month.

2. The method according to claim 1, wherein the glue plug-based substrate comprises coconut peat and a binder, and optionally perlite and/or peat.

3. The method according to claim 1 or 2, wherein the propagation medium is an MS medium supplemented with one or more auxins and one or more cytokinins, these cytokinins being selected from the group consisting of N6(Δ2-isopentenyl)adenine, trans-zeatin, dihydrozeatin, N6(benzyl)adenine, N6(benzyl)adenosine, 6-furfurylaminopurine, and thidiazuron, wherein the cytokinins other than thidiazuron are present at a concentration of 0.5-10 mg/L and thidiazuron is present at a concentration of 0.01-0.5 mg/L.

4. The method according to any of the previous claims 1-3, wherein the rooting medium contains auxins in a concentration of 0.1-5 mg/L, wherein the auxins are selected from naphthalene acetic acid (NAA) or indole butyric acid (IBA), wherein the rooting medium further contains KH₂PO₄, inositol and a sugar.

5. The method according to any of the previous claims 1-4, wherein the propagation medium and/or rooting medium is hardened with a gellan gum based gelling agent, wherein said gellan gum gelling agent is present at 1.6-2 g/L.

6. The method according to any of the previous claims 1-5, wherein both the propagation medium and the rooting medium further comprise adenine hemisulfate, biotin and folic acid.

7. The method according to any of the previous claims 1-6, wherein the juvenile bamboo plant at an age of 18 months has at least 10 leaves on the stems over a length of 10 cm measured from the substrate along the stems.

8. The method according to any of the previous claims 1-7, wherein the juvenile bamboo plant at an age of 18 months has at least 40 leaves on the stems over a length of 20 cm measured from the substrate along the stems.

9. The method according to any of the previous claims 1-8, wherein the rooted explant in the substrate and/or the juvenile bamboo plant in the greenhouse or grow room is watered with redox water at least twice a month.

10. The method according to any of the previous claims 1-9, wherein the glue plug-based substrate consists substantially of peat and a binder.

11. The method according to any of the previous claims 1-10, wherein the rooted explant is transplanted into the glue plug-based substrate when the plant is about 10 weeks old.
